(19) Europäisches Patentamt
European Patent Office
Office européen des brevets

(11) Veröffentlichungsnummer: **0 186 083**
**A2**

(12) # EUROPÄISCHE PATENTANMELDUNG

(21) Anmeldenummer: **85115997.0**

(22) Anmeldetag: **14.12.85**

(51) Int. Cl.[4]: **C 07 C 101/30**
C 07 C 125/065, C 07 C 99/00
A 61 K 31/195

(30) Priorität: **24.12.84 DE 3447295**

(43) Veröffentlichungstag der Anmeldung:
**02.07.86 Patentblatt 86/27**

(84) Benannte Vertragsstaaten:
**AT BE CH DE FR GB IT LI LU NL SE**

(71) Anmelder: **Asta-Werke Aktiengesellschaft Chemische Fabrik**
**Artur-Ladebeck-Strasse 128 - 152**
**D-4800 Bielefeld 14(DE)**

(72) Erfinder: **Lautenschläger, Hans-Heiner, Dr.**
**Neusser Gasse 50**
**D-5024 Pulheim(DE)**

(72) Erfinder: **Dereu, Norbert, Dr.**
**An der Holzhecke 11**
**D-5020 Frechen-Bachem(DE)**

(72) Erfinder: **Niemann, Reinhard, Dr.Dr.**
**Kurt-Schumacher Strasse 10**
**D-5060 Bergisch-Gladbach 1(DE)**

(72) Erfinder: **Hameister, Walter, Dr.**
**Tomburgstrasse 10**
**D-5024 Pulheim-Brauweiler(DE)**

(54) **Neue N-(2-Hydroxyalkyl)-aminosäuren und ihre Derivate, Verfahren zu ihrer Herstellung und diese enthaltende pharmazeutische Präparate.**

(57) Die vorliegende Erfindung betrifft neue N-(2-Hydroxyalkyl)-aminosäuren und ihre Derivate der allgemeinen Formeln I und II,

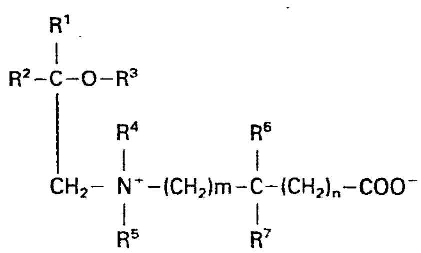

I

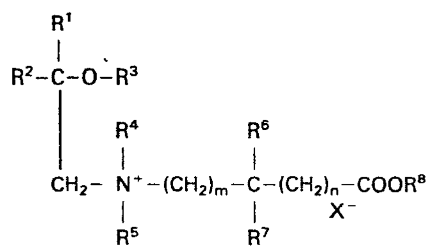

II

Verfahren zu ihrer Herstellung und diese enthaltende pharmazeutische Präparate.

EP 0 186 083 A2

ASTA-Werke Aktiengesellschaft, Chemische Fabrik
Artur-Ladebeck-Straße 128-152, 4800 Bielefeld 14

Titel: Neue N-(2-Hydroxyalkyl)-aminosäuren und ihre Derivate, Verfahren zu ihrer Herstellung und diese enthaltende pharmazeutische Präparate

## Beschreibung

Die vorliegende Erfindung betrifft neue N-(2-Hydroxyalkyl)-aminosäuren und ihre Derivate, die sich durch wertvolle pharmakologische Eigenschaften auszeichnen, sowie Verfahren zu ihrer Herstellung und ihre Verwendung als Wirkstoff in Arzneimitteln. Sie können insbesondere zur Behandlung von Pilzkrankheiten und Tumoren eingesetzt werden.

Die erfindungsgemäßen Verbindungen besitzen außerdem eine antimykotische und herbizide Wirkung.

Die erfindungsgemäßen Verbindungen entsprechen den allgemeinen Formeln I und II.

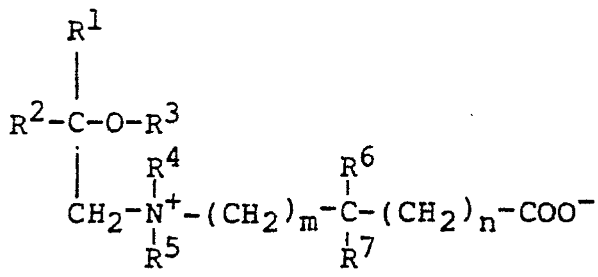 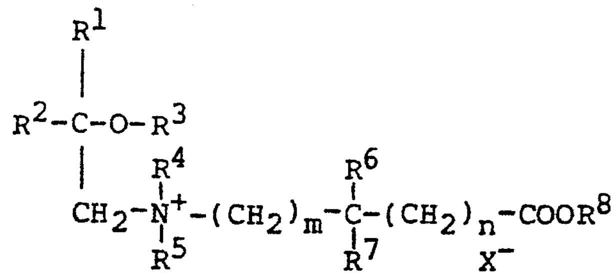

I II

worin

$R^1$ einen Alkylrest mit 10-20 Kohlenstoffatomen,

$R^2$ Wasserstoff oder einen Alkylrest mit 1-4 Kohlenstoffatomen und

$R^3$ Wasserstoff, einen gesättigten oder ungesättigten, geradkettigen oder verzweigten Alkylrest mit 1-20 Kohlenstoffatomen, einen Aralkylrest der Formel $-(CH_2)_p-$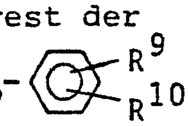

mit p gleich 1-4, $-CO-R^{11}$, $-COOR^{11}$, $-CO-NR^{11}R^{12}$, $-CS-NH-R^{11}$, $-O_2S-R^{11}$ bedeuten, während

$R^4, R^5$ gleich oder verschieden sind und für Wasserstoff, einen Alkylrest mit 1-4 Kohlenstoffatomen, Phenyl oder Benzyl,

$R^6, R^7$ gleich oder voneinander verschieden sind und für Wasserstoff, einen linearen oder verzweigten Alkylrest mit 1-4 Kohlenstoffatomen, einen Aralkylrest der Formel $-(CH_2)q-$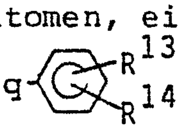

mit q gleich 0-4, eine Methylthioethyl-Gruppe und

$R^8$ für Wasserstoff oder einen Alkylrest mit 1-6 Kohlenstoffatomen stehen,

$R^9, R^{10}, R^{13}, R^{14}$ gleich oder voneinander verschieden sind und Wasserstoff, Halogen, einen Alkyl- oder Alkoxy-Rest mit 1-4 Kohlenstoffatomen, eine Trifluormethylgruppe oder

jeweils zusammen ($R^9 + R^{10}$; $R^{13} + R^{14}$) eine Methylendioxygruppe,

$R^{11}$, $R^{12}$ gleich oder voneinander verschieden sind und Wasserstoff, einen gesättigten oder ungesättigten, linearen oder verzweigten Alkylrest mit 1-19 Kohlenstoffatomen, einen Aralkylrest der Formel $-(CH_2)_r-$ 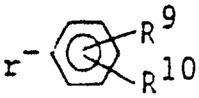 mit r gleich 0-4 bedeuten, während

$X^-$ ein Säureanion einer anorganischen oder organischen, ein- oder mehrbasischen Säure und die Summe aus

m + n eine ganze Zahl von 0-10 bedeuten.

Erfindungsgemäße Verbindungen sind beispielsweise:

N,N-Dimethyl-N-(2-hydroxydodecyl)-ammonioacetat
N,N-Dimethyl-N-(2-hydroxytridecyl)-ammonioacetat
N,N-Dimethyl-N-(2-hydroxytetradecyl)-ammonioacetat
N,N-Dimethyl-N-(2-hydroxypentadecyl)-ammonioacetat
N,N-Dimethyl-N-(2-hydroxyhexadecyl)-ammonioacetat
N,N-Dimethyl-N-(2-hydroxyheptadecyl)-ammonioacetat
N,N-Dimethyl-N-(2-hydroxyoctadecyl)-ammonioacetat
N,N-Dimethyl-N-(2-hydroxynonadecyl)-ammonioacetat
N,N-Dimethyl-N-(2-hydroxyeicosyl)-ammonioacetat
N,N-Dimethyl-N-(2-hydroxyheneicosyl)-ammonioacetat
N,N-Dimethyl-N-(2-hydroxydocosyl)-ammonioacetat
3-[N,N-Dimethyl-N-(2-hydroxydodecyl)-ammonio]-propionat
3-[N,N-Dimethyl-N-(2-hydroxytridecyl)-ammonio]-propionat
3-[N,N-Dimethyl-N-(2-hydroxytetradecyl)-ammonio]-propionat
3-[N,N-Dimethyl-N-(2-hydroxypentadecyl)-ammonio]-propionat
3-[N,N-Dimethyl-N-(2-hydroxyhexadecyl)-ammonio]-propionat
3-[N,N-Dimethyl-N-(2-hydroxyheptadecyl)-ammonio]-propionat
3-[N,N-Dimethyl-N-(2-hydroxyoctadecyl)-ammonio]-propionat
3-[N,N-Dimethyl-N-(2-hydroxynonadecyl)-ammonio]-propionat
3-[N,N-Dimethyl-N-(2-hydroxyeicosyl)-ammonio]-propionat
3-[N,N-Dimethyl-N-(2-hydroxyheneicosyl)-ammonio]-propionat
3-[N,N-Dimethyl-N-(2-hydroxydocosyl)-ammonio]-propionat

4-[N,N-Dimethyl-N-(2-hydroxydodecyl)-ammonio]-butyrat
4-[N,N-Dimethyl-N-(2-hydroxytridecyl)-ammonio]-butyrat
4-[N,N-Dimethyl-N-(2-hydroxytetradecyl)-ammonio]-butyrat
4-[N,N-Dimethyl-N-(2-hydroxypentadecyl)-ammonio]-butyrat
4-[N,N-Dimethyl-N-(2-hydroxyhexadecyl)-ammonio]-butyrat
4-[N,N-Dimethyl-N-(2-hydroxyheptadecyl)-ammonio]-butyrat
4-[N,N-Dimethyl-N-(2-hydroxyoctadecyl)-ammonio]-butyrat
4-[N,N-Dimethyl-N-(2-hydroxynonadecyl)-ammonio]-butyrat
4-[N,N-Dimethyl-N-(2-hydroxyeicosyl)-ammonio]-butyrat
4-[N,N-Dimethyl-N-(2-hydroxyheneicosyl)-ammonio]-butyrat
4-[N,N-Dimethyl-N-(2-hydroxydocosyl)-ammonio]-butyrat
5-[N,N-Dimethyl-N-(2-hydroxydodecyl)-ammonio]-pentanoat
5-[N,N-Dimethyl-N-(2-hydroxytridecyl)-ammonio]-pentanoat
5-[N,N-Dimethyl-N-(2-hydroxytetradecyl)-ammonio]-pentanoat
5-[N,N-Dimethyl-N-(2-hydroxypentadecyl)-ammonio]-pentanoat
5-[N,N-Dimethyl-N-(2-hydroxyhexadecyl)-ammonio]-pentanoat
5-[N,N-Dimethyl-N-(2-hydroxyheptadecyl)-ammonio]-pentanoat
5-[N,N-Dimethyl-N-(2-hydroxyoctadecyl)-ammonio]-pentanoat
5-[N,N-Dimethyl-N-(2-hydroxynonadecyl)-ammonio]-pentanoat
5-[N,N-Dimethyl-N-(2-hydroxyeicosyl)-ammonio]-pentanoat
5-[N,N-Dimethyl-N-(2-hydroxyheneicosyl)-ammonio]-pentanoat
5-[N,N-Dimethyl-N-(2-hydroxydocosyl)-ammonio]-pentanoat
6-[N,N-Dimethyl-N-(2-hydroxydodecyl)-ammonio]-hexanoat
6-[N,N-Dimethyl-N-(2-hydroxytridecyl)-ammonio]-hexanoat
6-[N,N-Dimethyl-N-(2-hydroxytetradecyl)-ammonio]-hexanoat
6-[N,N-Dimethyl-N-(2-hydroxypentadecyl)-ammonio]-hexanoat
6-[N,N-Dimethyl-N-(2-hydroxyhexadecyl)-ammonio]-hexanoat
6-[N,N-Dimethyl-N-(2-hydroxyheptadecyl)-ammonio]-hexanoat
6-[N,N-Dimethyl-N-(2-hydroxyoctadecyl)-ammonio]-hexanoat
6-[N,N-Dimethyl-N-(2-hydroxynonadecyl)-ammonio]-hexanoat
6-[N,N-Dimethyl-N-(2-hydroxyeicosyl)-ammonio]-hexanoat
6-[N,N-Dimethyl-N-(2-hydroxyheneicosyl)-ammonio]-hexanoat
6-[N,N-Dimethyl-N-(2-hydroxydocosyl)-ammonio]-hexanoat
7-[N,N-Dimethyl-N-(2-hydroxydodecyl)-ammonio]-heptanoat
7-[N,N-Dimethyl-N-(2-hydroxytridecyl)-ammonio]-heptanoat
7-[N,N-Dimethyl-N-(2-hydroxytetradecyl)-ammonio]-heptanoat
7-[N,N-Dimethyl-N-(2-hydroxypentadecyl)-ammonio]-heptanoat

7-[N,N-Dimethyl-N-(2-hydroxyhexadecyl)-ammonio]-heptanoat

7-[N,N-Dimethyl-N-(2-hydroxyheptadecyl)-ammonio]-heptanoat

7-[N,N-Dimethyl-N-(2-hydroxyoctadecyl)-ammonio]-heptanoat

7-[N,N-Dimethyl-N-(2-hydroxynonadecyl)-ammonio]-heptanoat

7-[N,N-Dimethyl-N-(2-hydroxyeicosyl)-ammonio]-heptanoat

7-[N,N-Dimethyl-N-(2-hydroxyheneicosyl)-ammonio]-heptanoat

7-[N,N-Dimethyl-N-(2-hydroxydocosyl)-ammonio]-heptanoat

8-[N,N-Dimethyl-N-(2-hydroxydodecyl)-ammonio]-octanoat

8-[N,N-Dimethyl-N-(2-hydroxytridecyl)-ammonio]-octanoat

8-[N,N-Dimethyl-N-(2-hydroxytetradecyl)-ammonio]-octanoat

8-[N,N-Dimethyl-N-(2-hydroxypentadecyl)-ammonio]-octanoat

8-[N,N-Dimethyl-N-(2-hydroxyhexadecyl)-ammonio]-octanoat

8-[N,N-Dimethyl-N-(2-hydroxyheptadecyl)-ammonio]-octanoat

8-[N,N-Dimethyl-N-(2-hydroxyoctadecyl)-ammonio]-octanoat

8-[N,N-Dimethyl-N-(2-hydroxynonadecyl)-ammonio]-octanoat

8-[N,N-Dimethyl-N-(2-hydroxyeicosyl)-ammonio]-octanoat

8-[N,N-Dimethyl-N-(2-hydroxyheneicosyl)-ammonio]-octanoat

8-[N,N-Dimethyl-N-(2-hydroxydocosyl)-ammonio]-octanoat

9-[N,N-Dimethyl-N-(2-hydroxydodecyl)-ammonio]-nonanoat

9-[N,N-Dimethyl-N-(2-hydroxytridecyl)-ammonio]-nonanoat

9-[N,N-Dimethyl-N-(2-hydroxytetradecyl)-ammonio]-nonanoat

9-[N,N-Dimethyl-N-(2-hydroxypentadecyl)-ammonio]-nonanoat

9-[N,N-Dimethyl-N-(2-hydroxyhexadecyl)-ammonio]-nonanoat

9-[N,N-Dimethyl-N-(2-hydroxyheptadecyl)-ammonio]-nonanoat

9-[N,N-Dimethyl-N-(2-hydroxyoctadecyl)-ammonio]-nonanoat

9-[N,N-Dimethyl-N-(2-hydroxynonadecyl)-ammonio]-nonanoat

9-[N,N-Dimethyl-N-(2-hydroxyeicosyl)-ammonio]-nonanoat

9-[N,N-Dimethyl-N-(2-hydroxyheneicosyl)-ammonio]-nonanoat

9-[N,N-Dimethyl-N-(2-hydroxydocosyl)-ammonio]-nonanoat

10-[N,N-Dimethyl-N-(2-hydroxydodecyl)-ammonio]-decanoat

10-[N,N-Dimethyl-N-(2-hydroxytridecyl)-ammonio]-decanoat

10-[N,N-Dimethyl-N-(2-hydroxytetradecyl)-ammonio]-decanoat

10-[N,N-Dimethyl-N-(2-hydroxypentadecyl)-ammonio]-decanoat

10-[N,N-Dimethyl-N-(2-hydroxyhexadecyl)-ammonio]-decanoat

10-[N,N-Dimethyl-N-(2-hydroxyheptadecyl)-ammonio]-decanoat

10-[N,N-Dimethyl-N-(2-hydroxyoctadecyl)-ammonio]-decanoat

10-[N,N-Dimethyl-N-(2-hydroxynonadecyl)-ammonio]-decanoat

10-[N,N-Dimethyl-N-(2-hydroxyeicosyl)-ammonio]-decanoat
10-[N,N-Dimethyl-N-(2-hydroxyheneicosyl)-ammonio]-decanoat
10-[N,N-Dimethyl-N-(2-hydroxydocosyl)-ammonio]-decanoat
11-[N,N-Dimethyl-N-(2-hydroxydodecyl)-ammonio]-undecanoat
11-[N,N-Dimethyl-N-(2-hydroxytridecyl)-ammonio]-undecanoat
11-[N,N-Dimethyl-N-(2-hydroxytetradecyl)-ammonio]-undecanoat
11-[N,N-Dimethyl-N-(2-hydroxypentadecyl)-ammonio]-undecanoat
11-[N,N-Dimethyl-N-(2-hydroxyhexadecyl)-ammonio]-undecanoat
11-[N,N-Dimethyl-N-(2-hydroxyheptadecyl)-ammonio]-undecanoat
11-[N,N-Dimethyl-N-(2-hydroxyoctadecyl)-ammonio]-undecanoat
11-[N,N-Dimethyl-N-(2-hydroxynonadecyl)-ammonio]-undecanoat
11-[N,N-Dimethyl-N-(2-hydroxyeicosyl)-ammonio]-undecanoat
11-[N,N-Dimethyl-N-(2-hydroxyheneicosyl)-ammonio]-undecanoat
11-[N,N-Dimethyl-N-(2-hydroxydocosyl)-ammonio]-undecanoat
12-[N,N-Dimethyl-N-(2-hydroxydodecyl)-ammonio]-dodecanoat
12-[N,N-Dimethyl-N-(2-hydroxytridecyl)-ammonio]-dodecanoat
12-[N,N-Dimethyl-N-(2-hydroxytetradecyl)-ammonio]-dodecanoat
12-[N,N-Dimethyl-N-(2-hydroxypentadecyl)-ammonio]-dodecanoat
12-[N,N-Dimethyl-N-(2-hydroxyhexadecyl)-ammonio]-dodecanoat
12-[N,N-Dimethyl-N-(2-hydroxyheptadecyl)-ammonio]-dodecanoat
12-[N,N-Dimethyl-N-(2-hydroxyoctadecyl)-ammonio]-dodecanoat
12-[N,N-Dimethyl-N-(2-hydroxynonadecyl)-ammonio]-dodecanoat
12-[N,N-Dimethyl-N-(2-hydroxyeicosyl)-ammonio]-dodecanoat

12-[N,N-Dimethyl-N-(2-hydroxyheneicosyl)-ammonio]-dodecanoat
12-[N,N-Dimethyl-N-(2-hydroxydocosyl)-ammonio]-dodecanoat

Zu den erfindungsgemäßen Verbindungen gehören analog den vorangenannten Verbindungen auch solche, in denen $R^2$ statt Wasserstoff eine Methyl-, Ethyl-, Propyl- oder Butylgruppe darstellt, z.B.:

N,N-Dimethyl-N-(2-hydroxy-2-methyldodecyl)-ammonioacetat
N,N-Dimethyl-N-(2-hydroxy-2-methyltridecyl)-ammonioacetat
N,N-Dimethyl-N-(2-hydroxy-2-methyltetradecyl)-ammonioacetat
N,N-Dimethyl-N-(2-hydroxy-2-methylpentadecyl)-ammonioacetat
N,N-Dimethyl-N-(2-hydroxy-2-methylhexadecyl)-ammonioacetat
N,N-Dimethyl-N-(2-hydroxy-2-methylheptadecyl)-ammonioacetat
N,N-Dimethyl-N-(2-hydroxy-2-methyloctadecyl)-ammonioacetat
N,N-Dimethyl-N-(2-hydroxy-2-methylnonadecyl)-ammonioacetat
N,N-Dimethyl-N-(2-hydroxy-2-methyleicosyl)-ammonioacetat
N,N-Dimethyl-N-(2-hydroxy-2-methylheneicosyl)-ammonioacetat
N,N-Dimethyl-N-(2-hydroxy-2-methyldocosyl)-ammonioacetat
3-[N,N-Dimethyl-N-(2-hydroxy-2-methyldodecyl)-ammonio]-propionat
3-[N,N-Dimethyl-N-(2-hydroxy-2-methyltridecyl)-ammonio]-propionat
3-[N,N-Dimethyl-N-(2-hydroxy-2-methyltetradecyl)-ammonio]-propionat
3-[N,N-Dimethyl-N-(2-hydroxy-2-methylpentadecyl)-ammonio]-propionat
3-[N,N-Dimethyl-N-(2-hydroxy-2-methylhexadecyl)-ammonio]-propionat
3-[N,N-Dimethyl-N-(2-hydroxy-2-methylheptadecyl)-ammonio]-propionat
3-[N,N-Dimethyl-N-(2-hydroxy-2-methyloctadecyl)-ammonio]-propionat

3-[N,N-Dimethyl-N-(2-hydroxy-2-methylnonadecyl)-ammonio]-propionat

3-[N,N-Dimethyl-N-(2-hydroxy-2-methyleicosyl)-ammonio]-propionat

3-[N,N-Dimethyl-N-(2-hydroxy-2-methylheneicosyl)-ammonio]-propionat

3-[N,N-Dimethyl-N-(2-hydroxy-2-methyldocosyl)-ammonio]-propionat

4-[N,N-Dimethyl-N-(2-hydroxy-2-methyldodecyl)-ammonio]-butyrat

4-[N,N-Dimethyl-N-(2-hydroxy-2-methyltridecyl)-ammonio]-butyrat

4-[N,N-Dimethyl-N-(2-hydroxy-2-methyltetradecyl)-ammonio]-butyrat

4-[N,N-Dimethyl-N-(2-hydroxy-2-methylpentadecyl)-ammonio]-butyrat

4-[N,N-Dimethyl-N-(2-hydroxy-2-methylhexadecyl)-ammonio]-butyrat

4-[N,N-Dimethyl-N-(2-hydroxy-2-methylheptadecyl)-ammonio]-butyrat

4-[N,N-Dimethyl-N-(2-hydroxy-2-methyloctadecyl)-ammonio]-butyrat

4-[N,N-Dimethyl-N-(2-hydroxy-2-methylnonadecyl)-ammonio]-butyrat

4-[N,N-Dimethyl-N-(2-hydroxy-2-methyleicosyl)-ammonio]-butyrat

4-[N,N-Dimethyl-N-(2-hydroxy-2-methylheneicosyl)-ammonio]-butyrat

4-[N,N-Dimethyl-N-(2-hydroxy-2-methyldocosyl)-ammonio]-butyrat

5-[N,N-Dimethyl-N-(2-hydroxy-2-methylhexadecyl)-ammonio]-pentanoat

5-[N,N-Dimethyl-N-(2-hydroxy-2-methyloctadecyl)-ammonio]-pentanoat

5-[N,N-Dimethyl-N-(2-hydroxy-2-methyleicosyl)-ammonio]-pentanoat

6-[N,N-Dimethyl-N-(2-hydroxy-2-methylhexadecyl)-ammonio]-hexanoat

6-[N,N-Dimethyl-N-(2-hydroxy-2-methyloctadecyl)-ammonio]-hexanoat

6-[N,N-Dimethyl-N-(2-hydroxy-2-methyleicosyl)-ammonio]-hexanoat

7-[N,N-Dimethyl-N-(2-hydroxy-2-methylhexadecyl)-ammonio]-heptanoat

7-[N,N-Dimethyl-N-(2-hydroxy-2-methyloctadecyl)-ammonio]-heptanoat

7-[N,N-Dimethyl-N-(2-hydroxy-2-methyleicosyl)-ammonio]-heptanoat

8-[N,N-Dimethyl-N-(2-hydroxy-2-methylhexadecyl)-ammonio]-octanoat

8-[N,N-Dimethyl-N-(2-hydroxy-2-methyloctadecyl)-ammonio]-octanoat

8-[N,N-Dimethyl-N-(2-hydroxy-2-methyleicosyl)-ammonio]-octanoat

9-[N,N-Dimethyl-N-(2-hydroxy-2-methylhexadecyl)-ammonio]-nonanoat

9-[N,N-Dimethyl-N-(2-hydroxy-2-methyloctadecyl)-ammonio]-nonanoat

9-[N,N-Dimethyl-N-(2-hydroxy-2-methyleicosyl)-ammonio]-nonanoat

10-[N,N-Dimethyl-N-(2-hydroxy-2-methylhexadecyl)-ammonio]-decanoat

10-[N,N-Dimethyl-N-(2-hydroxy-2-methyloctadecyl)-ammonio]-decanoat

10-[N,N-Dimethyl-N-(2-hydroxy-2-methyleicosyl)-ammonio]-decanoat

11-[N,N-Dimethyl-N-(2-hydroxy-2-methylhexadecyl)-ammonio]-undecanoat

11-[N,N-Dimethyl-N-(2-hydroxy-2-methyloctadecyl)-ammonio]-undecanoat

11-[N,N-Dimethyl-N-(2-hydroxy-2-methyleicosyl)-ammonio]-undecanoat

12-[N,N-Dimethyl-N-(2-hydroxy-2-methylhexadecyl)-ammonio]-dodecanoat

12-[N,N-Dimethyl-N-(2-hydroxy-2-methyloctadecyl)-ammonio]-dodecanoat

12-[N,N-Dimethyl-N-(2-hydroxy-2-methyleicosyl)-ammonio]-dodecanoat

N,N-Dimethyl-N-(2-ethyl-2-hydroxyhexadecyl)-ammonioacetat

N,N-Dimethyl-N-(2-ethyl-2-hydroxyoctadecyl)-ammonioacetat

N,N-Dimethyl-N-(2-ethyl-2-hydroxyeicosyl)-ammonioacetat

3-[N,N-Dimethyl-N-(2-ethyl-2-hydroxyhexadecyl)-ammonio]-propionat

3-[N,N-Dimethyl-N-(2-ethyl-2-hydroxyoctadecyl)-ammonio]-propionat

3-[N,N-Dimethyl-N-(2-ethyl-2-hydroxyeicosyl)-ammonio]-propionat

4-[N,N-Dimethyl-N-(2-ethyl-2-hydroxyhexadecyl)-ammonio]-butyrat

4-[N,N-Dimethyl-N-(2-ethyl-2-hydroxyoctadecyl)-ammonio]-butyrat

4-[N,N-Dimethyl-N-(2-ethyl-2-hydroxyeicosyl)-ammonio]-butyrat

5-[N,N-Dimethyl-N-(2-ethyl-2-hydroxyhexadecyl)-ammonio]-pentanoat

5-[N,N-Dimethyl-N-(2-ethyl-2-hydroxyoctadecyl)-ammonio]-pentanoat

5-[N,N-Dimethyl-N-(2-ethyl-2-hydroxyeicosyl)-ammonio]-pentanoat

6-[N,N-Dimethyl-N-(2-ethyl-2-hydroxyhexadecyl)-ammonio]-hexanoat

6-[N,N-Dimethyl-N-(2-ethyl-2-hydroxyoctadecyl)-ammonio]-hexanoat

6-[N,N-Dimethyl-N-(2-ethyl-2-hydroxyeicosyl)-ammonio]-hexanoat

N,N-Dimethyl-N-(2-hydroxy-2-propylhexadecyl)-ammonioacetat

N,N-Dimethyl-N-(2-hydroxy-2-propyloctadecyl)-ammonioacetat

N,N-Dimethyl-N-(2-hydroxy-2-propyleicosyl)-ammonioacetat

3-[N,N-Dimethyl-N-(2-hydroxy-2-propylhexadecyl)-ammonio]-propionat
3-[N,N-Dimethyl-N-(2-hydroxy-2-propyloctadecyl)-ammonio]-propionat
3-[N,N-Dimethyl-N-(2-hydroxy-2-propyleicosyl)-ammonio]-propionat
4-[N,N-Dimethyl-N-(2-hydroxy-2-propylhexadecyl)-ammonio]-butyrat
4-[N,N-Dimethyl-N-(2-hydroxy-2-propyloctadecyl)-ammonio]-butyrat
4-[N,N-Dimethyl-N-(2-hydroxy-2-propyleicosyl)-ammonio]-butyrat
N,N-Dimethyl-N-(2-butyl-2-hydroxyoctadecyl)-ammonioacetat
3-[N,N-Dimethyl-N-(2-butyl-2-hydroxyoctadecyl)-ammonio]-propionat
4-[N,N-Dimethyl-N-(2-butyl-2-hydroxyoctadecyl)-ammonio]-butyrat

Zu den erfindungsgemäßen Verbindungen gehören analog den vorangenannten Verbindungen auch solche, in denen die Reste $R^4$ und $R^5$ statt einer Methylgruppe Wasserstoff, eine Ethyl-, Propyl-, Butyl-, Phenyl- oder Benzylgruppe darstellen, wie z.B.

N-Ethyl-N-(2-hydroxyoctadecyl)-N-methyl-ammonioacetat
N-Benzyl-N-(2-hydroxyoctadecyl)-N-methyl-ammonioacetat
N-(2-Hydroxyoctadecyl)-N-methyl-N-propyl-ammonioacetat
N-Butyl-N-(2-hydroxyoctadecyl)-N-methyl-ammonioacetat
N-(2-Hydroxyoctadecyl)-N-methyl-ammonioacetat
N-(2-Hydroxyoctadecyl)-N-phenyl-ammonioacetat
N-(2-Hydroxyoctadecyl)-ammonioacetat
4-[N-Ethyl-N-(2-hydroxyoctadecyl)-N-methyl-ammonio]-butyrat
4-[N-Benzyl-N-(2-hydroxyoctadecyl)-N-methyl-ammonio]-butyrat
4-[N-(2-Hydroxyoctadecyl)-N-methyl-N-propyl-ammonio]-butyrat
4-[N-Butyl-N-(2-hydroxyoctadecyl)-N-methyl-ammonio]-butyrat
4-[N-(2-Hydroxyoctadecyl)-N-methyl-ammonio]-butyrat
4-[N-(2-Hydroxyoctadecyl)-N-phenyl-ammonio]-butyrat
4-[N-(2-Hydroxyoctadecyl)-ammonio]-butyrat

Zu den erfindungsgemäßen Verbindungen gehören analog den vorangenannten Verbindungen auch solche, in denen die Reste $R^6$ und $R^7$ unabhängig voneinander statt Wasserstoff Methyl-, Ethyl-, Isopropyl-, Isobutyl-, sec -Butyl-, n-Butyl-, Benzyl-, substituierte Benzyl-, 2-Phenylethyl-, substituierte 2-Phenylethyl-, 3-Phenylpropyl-, substituierte 3-Phenylpropyl-, 4-Phenylbutyl-, substituierte 4-Phenylbutyl-, Phenyl-, substituierte Phenyl- oder Methylthioethyl-Gruppen darstellen, wie z.B.

2-[N,N-Dimethyl-N-(2-hydroxyhexadecyl)-ammonio]-propionat
2-[N,N-Dimethyl-N-(2-hydroxyhexadecyl)-ammonio]-butyrat
2-[N,N-Dimethyl-N-(2-hydroxyhexadecyl)-ammonio]-3-methyl-butyrat
2-[N,N-Dimethyl-N-(2-hydroxyhexadecyl)-ammonio]-pentanoat
2-[N,N-Dimethyl-N-(2-hydroxyhexadecyl)-ammonio]-3-methyl-pentanoat
2-[N,N-Dimethyl-N-(2-hydroxyhexadecyl)-ammonio]-4-methyl-pentanoat
2-[N,N-Dimethyl-N-(2-hydroxyhexadecyl)-ammonio]-hexanoat
2-[N,N-Dimethyl-N-(2-hydroxyhexadecyl)-ammonio]-4-methyl-thio-butyrat
2-[N,N-Dimethyl-N-(2-hydroxyhexadecyl)-ammonio]-3-phenyl-propionat
2-[N,N-Dimethyl-N-(2-hydroxyhexadecyl)-ammonio]-3-(4-hydroxyphenyl)-propionat
4-[N,N-Dimethyl-N-(2-hydroxyhexadecyl)-ammonio]-2,2-dimethyl-butyrat
6-[N,N-Dimethyl-N-(2-hydroxyhexadecyl)-ammonio]-2,2-dimethyl-hexanoat
2-[N,N-Dimethyl-N-(2-hydroxyoctadecyl)-ammonio]-propionat
2-[N,N-Dimethyl-N-(2-hydroxyoctadecyl)-ammonio]-butyrat
2-[N,N-Dimethyl-N-(2-hydroxyoctadecyl)-ammonio]-3-methyl-butyrat
2-[N,N-Dimethyl-N-(2-hydroxyoctadecyl)-ammonio]-pentanoat
2-[N,N-Dimethyl-N-(2-hydroxyoctadecyl)-ammonio]-3-methyl-pentanoat

1

2-[N,N-Dimethyl-N-(2-hydroxyoctadecyl)-ammonio]-4-methyl-pentanoat

2-[N,N-Dimethyl-N-(2-hydroxyoctadecyl)-ammonio]-hexanoat

2-[N,N-Dimethyl-N-(2-hydroxyoctadecyl)-ammonio]-4-methyl-thio-butyrat

2-[N,N-Dimethyl-N-(2-hydroxyoctadecyl)-ammonio]-3-phenyl-propionat

2-[N,N-Dimethyl-N-(2-hydroxyoctadecyl)-ammonio]-3-(4-hydroxyphenyl)-propionat

4-[N,N-Dimethyl-N-(2-hydroxyoctadecyl)-ammonio]-2,2-dimethyl-butyrat

6-[N,N-Dimethyl-N-(2-hydroxyoctadecyl)-ammonio]-2,2-dimethyl-hexanoat

2-[N,N-Dimethyl-N-(2-hydroxyoctadecyl)-ammonio]-2-phenyl-propionat

Zu den erfindungsgemäßen Verbindungen gehören analog den vorangenannten Verbindungen auch solche, in denen $R^3$ statt Wasserstoff z.B. eine Alkyl-, Alkenyl-, Aralkyl-, Acyl-, Alkoxycarbonyl-, Alkenoxycarbonyl-, Aryloxycarbonyl-, Arylalkoxycarbonyl-, Alkylcarbamoyl-, Alkenylcarbamoyl-, Arylcarbamoyl-, Arylalkylcarbamoyl-, Alkylthiocarbamoyl-, Alkenylthiocarbamoyl-, Arylthiocarbamoyl-, Arylalkylthiocarbamoyl-, Alkylsulfonyl-, Alkenylsulfonyl-, Arylsulfonyl- oder Arylalkylsulfonyl-Gruppe darstellt, wie z.B.

N,N-Dimethyl-N-(2-methoxyhexadecyl)-ammonioacetat

4-[N,N-Dimethyl-N-(2-methoxyhexadecyl)-ammonio]-butyrat

N,N-Dimethyl-N-(2-methoxyoctadecyl)-ammonioacetat

4-[N,N-Dimethyl-N-(2-methoxyoctadecyl)-ammonio]-butyrat

N,N-Dimethyl-N-(2-methoxyeicosyl)-ammonioacetat

4-[N,N-Dimethyl-N-(2-methoxyeicosyl)-ammonio]-butyrat

N,N-Dimethyl-N-(2-ethoxyhexadecyl)-ammonioacetat

4-[N,N-Dimethyl-N-(2-ethoxyhexadecyl)-ammonio]-butyrat

N,N-Dimethyl-N-(2-ethoxyoctadecyl)-ammonioacetat

4-[N,N-Dimethyl-N-(2-ethoxyoctadecyl)-ammonio]-butyrat
N,N-Dimethyl-N-(2-ethoxyeicosyl)-ammonioacetat
4-[N,N-Dimethyl-N-(2-ethoxyeicosyl)-ammonio]-butyrat
N-(2-Allyloxyoctadecyl)-N,N-dimethyl-ammonioacetat
4-[N-(2-Allyloxyoctadecyl)-N,N-dimethyl-ammonio]-butyrat
N-(2-Hexadecyloxyoctadecyl)-N,N-dimethyl-ammonioacetat
4-[N-(2-Hexadecyloxyoctadecyl)-N,N-dimethyl-ammonio]-butyrat
N-(2-Benzyloxyoctadecyl)-N,N-dimethyl-ammonioacetat
4-[N-(2-Benzyloxyoctadecyl)-N,N-dimethyl-ammonio]-butyrat
N-(2-Acetoxyhexadecyl)-N,N-dimethyl-ammonioacetat
3-[N-(2-Acetoxyhexadecyl)-N,N-dimethyl-ammonio]-propionat
4-[N-(2-Acetoxyhexadecyl)-N,N-dimethyl-ammonio]-butyrat
5-[N-(2-Acetoxyhexadecyl)-N,N-dimethyl-ammonio]-pentanoat
6-[N-(2-Acetoxyhexadecyl)-N,N-dimethyl-ammonio]-hexanoat
N-(2-Acetoxyoctadecyl)-N,N-dimethyl-ammonioacetat
3-[N-(2-Acetoxyoctadecyl)-N,N-dimethyl-ammonio]-propionat
4-[N-(2-Acetoxyoctadecyl)-N,N-dimethyl-ammonio]-butyrat
5-[N-(2-Acetoxyoctadecyl)-N,N-dimethyl-ammonio]-pentanoat
6-[N-(2-Acetoxyoctadecyl)-N,N-dimethyl-ammonio]-hexanoat
N-(2-Acetoxyeicosyl)-N,N-dimethyl-ammonioacetat
3-[N-(2-Acetoxyeicosyl)-N,N-dimethyl-ammonio]-propionat
4-[N-(2-Acetoxyeicosyl)-N,N-dimethyl-ammonio]-butyrat
5-[N-(2-Acetoxyeicosyl)-N,N-dimethyl-ammonio]-pentanoat
6-[N-(2-Acetoxyeicosyl)-N,N-dimethyl-ammonio]-hexanoat
N,N-Dimethyl-N-(2-propionyloxyhexadecyl)-ammonioacetat
3-[N,N-Dimethyl-N-(2-propionyloxyhexadecyl)-ammonio]-propionat
4-[N,N-Dimethyl-N-(2-propionyloxyhexadecyl)-ammonio]-butyrat
5-[N,N-Dimethyl-N-(2-propionyloxyhexadecyl)-ammonio]-pentanoat
6-[N,N-Dimethyl-N-(2-propionyloxyhexadecyl)-ammonio]-hexanoat
N,N-Dimethyl-N-(2-propionyloxyoctadecyl)-ammonioacetat
3-[N,N-Dimethyl-N-(2-propionyloxyoctadecyl)-ammonio]-propionat

4-[N,N-Dimethyl-N-(2-propionyloxyoctadecyl)-ammonio]-butyrat
5-[N,N-Dimethyl-N-(2-propionyloxyoctadecyl)-ammonio]-pentanoat
6-[N,N-Dimethyl-N-(2-propionyloxyoctadecyl)-ammonio]-hexanoat
N,N-Dimethyl-N-(2-propionyloxyeicosyl)-ammonioacetat
3-[N,N-Dimethyl-N-(2-propionyloxyeicosyl)-ammonio]-propionat
4-[N,N-Dimethyl-N-(2-propionyloxyeicosyl)-ammonio]-butyrat
5-[N,N-Dimethyl-N-(2-propionyloxyeicosyl)-ammonio]-pentanoat
6-[N,N-Dimethyl-N-(2-propionyloxyeicosyl)-ammonio]-hexanoat
N,N-Dimethyl-N-(2-palmitoyloxyhexadecyl)-ammonioacetat
4-[N,N-Dimethyl-N-(2-palmitoyloxyhexadecyl)-ammonio]-butyrat
N,N-Dimethyl-N-(2-palmitoyloxyoctadecyl)-ammonioacetat
4-[N,N-Dimethyl-N-(2-palmitoyloxyoctadecyl)-ammonio]-butyrat
N,N-Dimethyl-N-(2-palmitoyloxyeicosyl)-ammonioacetat
4-[N,N-Dimethyl-N-(2-palmitoyloxyeicosyl)-ammonio]-butyrat
N,N-Dimethyl-N-(2-stearoyloxyhexadecyl)-ammonioacetat
4-[N,N-Dimethyl-N-(2-stearoyloxyhexadecyl)-ammonio]-butyrat
N,N-Dimethyl-N-(2-stearoyloxyoctadecyl)-ammonioacetat
4-[N,N-Dimethyl-N-(2-stearoyloxyoctadecyl)-ammonio]-butyrat
N,N-Dimethyl-N-(2-stearoyloxyeicosyl)-ammonioacetat
4-[N,N-Dimethyl-N-(2-stearoyloxyeicosyl)-ammonio]-butyrat
N,N-Dimethyl-N-(2-oleoyloxyhexadecyl)-ammonioacetat
4-[N,N-Dimethyl-N-(2-oleoyloxyhexadecyl)-ammonio]-butyrat
N,N-Dimethyl-N-(2-oleoyloxyoctadecyl)-ammonioacetat
4-[N,N-Dimethyl-N-(2-oleoyloxyoctadecyl)-ammonio]-butyrat
N,N-Dimethyl-N-(2-oleoyloxyeicosyl)-ammonioacetat
4-[N,N-Dimethyl-N-(2-oleoyloxyeicosyl)-ammonio]-butyrat
N,N-Dimethyl-N-(2-linoloyloxyhexadecyl)-ammonioacetat

4-[N,N-Dimethyl-N-(2-linoloyloxyhexadecyl)-ammonio]-butyrat

N,N-Dimethyl-N-(2-linoloyloxyoctadecyl)-ammonioacetat

4-[N,N-Dimethyl-N-(2-linoloyloxyoctadecyl)-ammonio]-butyrat

N,N-Dimethyl-N-(2-linoloyloxyeicosyl)-ammonioacetat

4-[N,N-Dimethyl-N-(2-linoloyloxyeicosyl)-ammonio]-butyrat

4-[N-(2-Benzoyloxyoctadecyl)-N,N-dimethyl-ammonio]-butyrat

4-[N-(2-Benzyloxycarbonyloxyoctadecyl)-N,N-dimethyl-ammonio]-butyrat

4-[N,N-Dimethyl-N-(2-methoxycarbonyloxyoctadecyl)-ammonio]-butyrat

4-[N,N-Dimethyl-N-(2-ethoxycarbonyloxyoctadecyl)-ammonio]-butyrat

4-[N,N-Dimethyl-N-(2-oleoyloxycarbonyloxyoctadecyl)-ammonio]-butyrat

N,N-Dimethyl-N-(2-methylcarbamoyloxyhexadecyl)-ammonioacetat

4-[N,N-Dimethyl-N-(2-methylcarbamoyloxyhexadecyl)-ammonio]-butyrat

N,N-Dimethyl-N-(2-methylcarbamoyloxyoctadecyl)-ammonioacetat

4-[N,N-Dimethyl-N-(2-methylcarbamoyloxyoctadecyl)-ammonio]-butyrat

N,N-Dimethyl-N-(2-methylcarbamoyloxyeicosyl)-ammonioacetat

4-[N,N-Dimethyl-N-(2-methylcarbamoyloxyeicosyl)-ammonio]-butyrat

N,N-Dimethyl-N-(2-ethylcarbamoyloxyhexadecyl)-ammonioacetat

4-[N,N-Dimethyl-N-(2-ethylcarbamoyloxyhexadecyl)-ammonio]-butyrat

N,N-Dimethyl-N-(2-ethylcarbamoyloxyoctadecyl)-ammonioacetat

4-[N,N-Dimethyl-N-(2-ethylcarbamoyloxyoctadecyl)-ammonio]-butyrat

N,N-Dimethyl-N-(2-ethylcarbamoyloxyeicosyl)-ammonioacetat

4-[N,N-Dimethyl-N-(2-ethylcarbamoyloxyeicosyl)-ammonio]-butyrat

4-[N-(2-Allylcarbamoyloxyoctadecyl-N,N-dimethyl)-ammonio]-butyrat

4-[N,N-Dimethyl-N-(2-dimethylcarbamoyloxyoctadecyl)-ammonio]-butyrat

4-[N-(2-Benzylcarbamoyloxyoctadecyl-N,N-dimethyl)-ammonio]-butyrat

4-[N,N-Dimethyl-N-(2-hexadecylcarbamoyloxyoctadecyl)-ammonio]-butyrat

4-[N,N-Dimethyl-N-(2-phenylcarbamoyloxyoctadecyl)-ammonio]-butyrat

4-[N,N-Dimethyl-N-(2-methylthiocarbamoyloxyoctadecyl)-ammonio]-butyrat

4-[N,N-Dimethyl-N-(2-ethylthiocarbamoyloxyoctadecyl)-ammonio]-butyrat

4-[N,N-Dimethyl-N-(2-phenylthiocarbamoyloxyoctadecyl)-ammonio]-butyrat

4-[N-(2-Benzylthiocarbamoyloxyoctadecyl-N,N-dimethyl)-ammonio]-butyrat

4-[N,N-Dimethyl-N-(2-methylsulfonyloxyoctadecyl)-ammonio]-butyrat

4-[N,N-Dimethyl-N-(2-p-tolylsulfonyloxyoctadecyl)-ammonio]-butyrat

4-[N-(2-Benzylsulfonyloxyoctadecyl)-N,N-dimethyl-ammonio]-butyrat

4-[N-(2-Allylsulfonyloxyoctadecyl)-N,N-dimethyl-ammonio]-butyrat

Zu den erfindungsgemäßen Verbindungen gehören analog den vorangenannten Verbindungen der Formel I auch solche der Formel II, in der $R^8$ ein Wasserstoffatom, einen Alkylrest mit 1-6 Kohlenstoffatomen, wie z.B. Methyl, Ethyl, Propyl, Isopropyl, Butyl, Pentyl, Hexyl und $X^-$ ein Säureanion einer anorganischen oder organischen, ein- oder mehrbasischen Säure darstellt, wie z.B. Chlorid, Sulfat, Phosphat, Acetat, Succinat, Lactat, Maleat, Malonat, Citrat, Fumarat. Beispiele für Verbindungen der Formel II sind:

N,N-Dimethyl-N-3-ethoxycarbonylpropyl-N-2-methylcarbamoyloxyoctadecyl-ammoniumchlorid

Bis-(N,N-dimethyl-N-5-isopropoxycarbonylpentyl-N-2-methoxyeicosyl-ammonium)-sulfat

N-Ethoxycarbonylmethyl-N-methyl-N-2-methoxyoctadecyl-ammoniumfumarat.

Verbindungen der Formel II werden hergestellt, indem man Epoxide der allgemeinen Formel III,

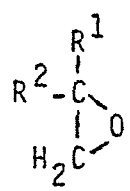

III

in der $R^1$ und $R^2$ die in den Formeln I und II angegebenen Bedeutungen haben, mit Aminen der Formel IV,

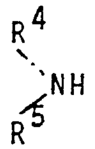

IV

in der $R^4$ und $R^5$ die in den Formeln I und II angegebenen Bedeutungen besitzen, in organischen oder organisch-wässrigen Lösungsmittelsystemen, bevorzugt Methanol, Ethanol, gegebenenfalls unter Druck, zu den ß-Aminoalkoholen der Formel V,

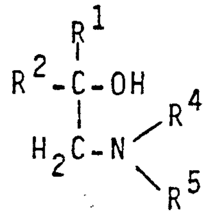

V

in der $R^1$, $R^2$, $R^4$, $R^5$ die in den Formeln I und II angegebenen Bedeutungen haben, umsetzt und diese mit einem Alkylierungsmittel der Formel VI,

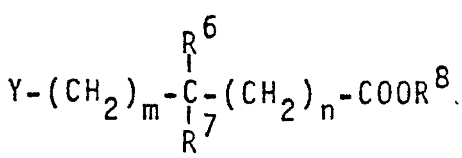

VI

in der $R^6$, $R^7$, $R^8$, m und n die in den Formeln I und II angegebenen Bedeutungen haben und Y ein Chlor-, Brom-, Jodatom oder eine ähnliche Abgangsgruppe darstellt, in einem indifferenten organischen Lösungsmittel, wie z.B. Acetonitril, Aceton bei Raum- bis Rückflußtemperatur behandelt, wobei Verbindungen der Formel II mit $R^3 = H$ entstehen.

Als Ausgangssubstanzen der Formel III kommen in Frage: 1,2-Epoxydodecan, 1,2-Epoxytridecan, 1,2-Epoxytetradecan, 1,2-Epoxypentadecan, 1,2-Epoxyhexadecan, 1,2-Epoxyheptadecan, 1,2-Epoxyoctadecan, 1,2-Epoxynonadecan, 1,2-Epoxyeicosan, 1,2-Epoxyheneicosan, 1,2-Epoxydocosan, 2-Methyl-1,2-epoxydodecan, 2-Methyl-1,2-epoxytridecan, 2-Methyl-1,2-epoxytetradecan, 2-Methyl-1,2-epoxypentadecan, 2-Methyl-1,2-epoxyhexadecan, 2-Methyl-1,2-epoxyheptadecan, 2-Methyl-1,2-epoxyoctadecan, 2-Methyl-1,2-epoxynonadecan, 2-Methyl-1,2-epoxyeicosan, 2-Methyl-1,2-epoxyheneicosan, 2-Methyl-1,2-epoxydocosan, 1,2-Epoxy-2-ethyldodecan, 1,2-Epoxy-2-ethyltridecan, 1,2-Epoxy-2-ethyltetradecan, 1,2-Epoxy-2-ethylpentadecan, 1,2-Epoxy-2-ethylhexadecan, 1,2-Epoxy-2-ethylheptadecan, 1,2-Epoxy-2-ethyloctadecan, 1,2-Epoxy-2-ethylnonadecan, 1,2-Epoxy-2-ethyleicosan, 1,2-Epoxy-2-ethylheneicosan, 1,2-Epoxy-2-ethyldocosan, 1,2-Epoxy-2-propyldodecan, 1,2-Epoxy-2-propyltridecan, 1,2-Epoxy-2-propyltetradecan, 1,2-Epoxy-2-propylpentadecan, 1,2-Epoxy-2-propylhexadecan, 1,2-Epoxy-2-propylheptadecan, 1,2-Epoxy-2-propyloctadecan, 1,2-Epoxy-2-propylnonadecan, 1,2-Epoxy-2-propyleicosan, 1,2-Epoxy-2-propylheneicosan, 1,2-Epoxy-2-propyldocosan, 2-Butyl-1,2-epoxydodecan, 2-Butyl-1,2-epoxytridecan, 2-Butyl-1,2-epoxytetradecan, 2-Butyl-1,2-epoxypentadecan, 2-Butyl-1,2-epoxyhexadecan, 2-Butyl-1,2-epoxyheptadecan, 2-Butyl-1,2-epoxyoctadecan, 2-Butyl-1,2-epoxynonadecan, 2-Butyl-1,2-epoxyeicosan, 2-Butyl-1,2-epoxyheneicosan, 2-Butyl-1,2-epoxydocosan.

Als Ausgangssubstanzen der Formel IV kommen z.B. in Frage: Methylamin, Ethylamin, Propylamin, Isopropylamin, Butylamin, Anilin, Benzylamin, Dimethylamin, Diethylamin, Dipropylamin, Dibutylamin, N-Methyl-ethylamin, N-Methyl-propylamin, N-Methyl-butylamin, N-Methylanilin, N-Methylbenzylamin, N-Ethyl-propylamin, N-Ethyl-isopropylamin, N-Ethyl-butylamin, N-Ethylanilin, N-Ethyl-benzylamin, N-Isopropyl-propylamin, N-Propyl-butylamin, N-Propylanilin,

N-Propyl-benzylamin, N-Butylanilin, N-Butyl-benzylamin.

Als Alkylierungsmittel der Formel VI kommen z.B. die Methyl-, Ethyl-, Propyl-, Isopropyl-, Butyl-, Pentyl-, Hexylester folgender Säuren in Frage:
Chloressigsäure, Bromessigsäure, 2-Chlorpropionsäure, 2-Brompropionsäure, 3-Chlorpropionsäure, 3-Brompropionsäure, 2-Chlorbuttersäure, 2-Brombuttersäure, 4-Chlorbuttersäure, 4-Brombuttersäure, 2-Chlorvaleriansäure, 2-Bromvaleriansäure, 5-Chlorvaleriansäure, 5-Bromvaleriansäure, 2-Chlorcapronsäure, 2-Bromcapronsäure, 6-Chlorcapronsäure, 6-Bromcapronsäure, 7-Chlorönanthsäure, 7-Bromönanthsäure, 8-Chlorcaprylsäure, 8-Bromcaprylsäure, 9-Chlorpelargonsäure, 9-Brompelargonsäure, 10-Chlorcaprinsäure, 10-Bromcaprinsäure, 11-Chlorundecansäure, 11-Bromundecansäure, 12-Chlordodecansäure, 12-Bromdodecansäure, 2-Chlor-3-methylbuttersäure, 2-Brom-3-methylbuttersäure, 2-Chlor-4-methylvaleriansäure, 2-Brom-4-methylvaleriansäure, 2-Chlor-3-methylvaleriansäure, 2-Brom-3-methylvaleriansäure, 2-Chlor-2-phenyl-propionsäure, 2-Brom-2-phenyl-propionsäure, 2-Chlor-3-phenyl-propionsäure, 2-Brom-3-phenyl-propionsäure, 2-Chlor-2-phenylessigsäure, 2-Brom-2-phenylessigsäure, 2-Chlor-4-methylthio-buttersäure, 2-Brom-4-methylthio-buttersäure, 3-Chlor-2-methylpropionsäure, 3-Brom-2-methylpropionsäure, 4-Chlor-2-methylbuttersäure, 4-Brom-2-methylbuttersäure, 5-Chlor-2-methylvaleriansäure, 5-Brom-2-methylvaleriansäure, 6-Chlor-2-methylcapronsäure, 6-Brom-2-methylcapronsäure, 7-Chlor-2-methylönanthsäure, 7-Brom-2-methylönanthsäure, 8-Chlor-2-methylcaprylsäure, 8-Brom-2-methylcaprylsäure, 9-Chlor-2-methylpelargonsäure, 9-Brom-2-methylpelargonsäure, 10-Chlor-2-methylcaprinsäure, 10-Brom-2-methylcaprinsäure, 11-Chlor-2-methylundecansäure, 11-Brom-2-methylundecansäure, 12-Chlor-2-methyldodecansäure, 12-Brom-2-methyldodecansäure, 3-Chlor-2,2-dimethylpropionsäure, 3-Brom-2,2-dimethylpropionsäure, 4-Chlor-2,2-dimethylbuttersäure, 4-Brom-2,2-dimethylbuttersäure, 5-Chlor-2,2-dimethyl-valeriansäure, 5-Brom-2,2-dimethyl-valeriansäure, 6-Chlor-2,2-dimethylcapronsäure, 6-Brom-2,2-dimethylcapronsäure, 7-Chlor-2,2-dimethylönanthsäure, 7-Brom-2,2-dimethylönanthsäure, 8-Chlor-2,2-dimethylcaprylsäure, 8-Brom-2,2-dimethylcaprylsäure, 9-Chlor-2,2-dimethylpelargonsäure, 9-Brom-2,2-dimethylpelargonsäure, 10-Chlor-2,2-dimethylcaprinsäure, 10-Brom-2,2-dimethyl-caprinsäure, 11-Chlor-2,2-dimethylundecansäure, 11-Brom-2,2-dimethylundecansäure, 12-Chlor-2,2-dimethyldodecan-säure, 12-Brom-2,2-dimethyldodecansäure.

Verbindungen der Formel II können auch hergestellt werden, indem man Epoxide der allgemeinen Formel III mit Aminocarbonsäureestern der allgemeinen Formel VII,

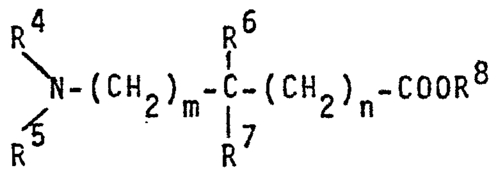

VII

in der $R^4$, $R^5$, $R^6$, $R^7$, $R^8$, m und n die in den Formeln I und II angegebenen Bedeutungen haben, in organischen oder organisch-wässrigen Lösungsmittelsystemen, bevorzugt Methanol oder Ethanol, gegebenenfalls unter Druck umsetzt und nachfolgend mit einer Säure der Formel HX, deren Anion $X^-$ die in Formel II angegebene Bedeutung hat, behandelt.

Als Ausgangsverbindungen der Formel VII kommen z.B. die Methyl-, Ethyl-, Propyl-, Isopropyl-, Butyl-, Pentyl-, Hexylester folgender Säuren in Frage:
Aminoessigsäure, 2-Aminopropionsäure, 3-Aminopropionsäure, 2-Aminobuttersäure, 4-Aminobuttersäure, 2-Aminovaleriansäure, 5-Aminovaleriansäure, 2-Aminocapronsäure,

6-Aminocapronsäure, 2-Aminoönanthsäure, 7-Aminoönanthsäure, 2-Aminocaprylsäure, 8-Aminocaprylsäure, 2-Aminopelargonsäure, 9-Aminopelargonsäure, 2-Aminocaprinsäure, 10-Aminocaprinsäure, 2-Aminoundecansäure, 11-Aminoundecansäure, 2-Aminododecansäure, 12-Aminododecansäure, Valin, Leucin, Isoleucin, Phenylalanin, Methionin, 2-Amino-2-phenylpropionsäure, 2-Amino-2-phenylessigsäure, 3-Amino-2-methylpropionsäure, 4-Amino-2-methylbuttersäure, 5-Amino-2-methylvaleriansäure, 6-Amino-2-methylcapronsäure, 7-Amino-2-methylönanthsäure, 8-Amino-2-methylcaprylsäure, 9-Amino-2-methylpelargonsäure, 10-Amino-2-methylcaprinsäure, 11-Amino-2-methyl-undecansäure, 12-Amino-2-methyldodecansäure, 3-Amino-2,2-dimethylpropionsäure, 4-Amino-2,2-dimethylbuttersäure, 5-Amino- 2,2-dimethylvaleriansäure, 6-Amino-2,2-dimethylcapronsäure, 7-Amino-2,2-dimethylönanthsäure, 8-Amino-2,2- dimethylcaprylsäure, 9-Amino-2,2-dimethylpelargonsäure, 10-Amino-2,2-dimethylcaprinsäure, 11-Amino-2,2-dimethylundecansäure, 12-Amino-2,2-dimethyldodecansäure sowie die N-Methyl-, N-Ethyl-, N-Propyl-, N-Isopropyl-, N-Butyl-, N-PHenyl- und N-Benzyl-Derivate der genannten Ester.

Verbindungen der Formel I und II mit $R^3$ ungleich Wasserstoff werden hergestellt, indem man Verbindungen der Formel I und II mit $R^3$ = Wasserstoff und $R^4$, $R^5$ ungleich Wasserstoff mit Alkylierungsmitteln oder Acylierungsmitteln der Formel VIII,

$$Z - R^3 \qquad \text{VIII}$$

worin $R^3$ die in den Formeln I und II angegebene Bedeutung hat (mit Ausnahme von Wasserstoff) und Z ein Halogenatom, Tosyloxy-, Acyloxy-Rest oder eine ähnliche Abgangsgruppe darstellt, in Substanz oder in einem indifferenten organischen Lösungsmittel, gegebenenfalls unter Druck und Anwendung einer Hilfsbase, die mit dem Acylierungsmittel oder Alkylierungsmittel und dem Rest $R^8$ compatibel ist, um-

setzt, wobei als Acylierungsmittel sinngemäß auch organisch Isocyanate oder Isothiocyanate der Formeln IX und X in Frage kommen,

$$R^{11}-N=C=O \qquad\qquad R^{11}-N=C=S$$

IX X

in denen $R^{11}$ die in den Formeln I und II angegebene Bedeutung hat.

Als Alkylierungsmittel der Formel VIII können z.B. eingesetzt werden:
Diazomethan, Dimethylsulfat, Chlormethan, Brommethan, Jodmethan, Chlorethan, Bromethan, Jodethan, Chlorpropan, Brompropan, Jodpropan, Allylchlorid, Allylbromid, Chlorbutan, Brombutan, Jodbutan, Chlorpentan, Brompentan, Jodpentan, Chlorhexan, Bromhexan, Jodhexan, 6-Brom-1-hexen, Chlorheptan, Bromheptan, Jodheptan, Chloroctan, Bromoctan, Jodoctan, p-Toluolsulfonsäureoctylester, Chlornonan, Bromnonan, Jodnonan, Chlordecan, Bromdecan, Joddecan, p-Toluolsulfonsäuredecylester, Chlorundecan, Bromundecan, Jodundecan, Chlordodecan, Bromdodecan, Joddodecan, p-Toluolsulfonsäuredodecylester, Chlortridecan, Bromtridecan, Jodtridecan, Chlortetradecan, Bromtetradecan, Jodtetradecan, p-Toluolsulfonsäuretetradecylester, Chlorpentadecan, Brompentadecan, Jodpentadecan, Chlorhexadecan, Bromhexadecan, Jodhexadecan, p-Toluolsulfonsäurehexadecylester, Methansulfonsäurehexadecylester, Chlorheptadecan, Bromheptadecan, Jodheptadecan, Chloroctadecan, Bromoctadecan, Jodoctadecan, p-Toluolsulfonsäureoctadecylester, 1-Chlor-9-octadecen, 1-Brom-9-octadecen, 1-Jod-9-octadecen, Chlornonadecan, Bromnonadecan, Jodnonadecan, Chloreicosan, Bromeicosan, Jodeicosan, p-Toluolsulfonsäureeicosylester, Benzylchlorid, Benzylbromid, 3-Chlorpropylbenzol, 3-Brompropylbenzol, 3-Jodpropylbenzol, 4-Chlorbutylbenzol, 4-Brombutylbenzol, 4-Jodbutylbenzol, p-Toluolsulfonsäure-4-phenylbutylester, wobei Chlor- und Bromverbindungen zweckmäßigerweise in Gegenwart von Alkalijodiden, insbesondere Natriumjodid, angewendet werden.

Als Acylierung der Formel VIII kommen z.B. die folgenden Säurechloride und -anhydride in Frage:
Essigsäurechlorid, -anhydrid, Propionsäurechlorid, -anhydrid, Buttersäurechlorid, -anhydrid, Valeriansäurechlorid, -anhydrid, Isovaleriansäurechlorid, -anhydrid, Capronsäurechlorid, -anhydrid, Önanthsäurechlorid, -anhydrid, Caprylsäurechlorid, -anhydrid, Pelargonsäurechlorid, -anhydrid, Caprinsäurechlorid, -anhydrid, Undecansäurechlorid, -anhydrid, Dodecansäurechlorid, -anhydrid, Tridecansäurechlorid, -anhydrid, Tetradecansäurechlorid, -anhydrid, Pentadecansäurechlorid, -anhydrid, Hexadecansäurechlorid, -anhydrid, Heptadecansäurechlorid, -anhydrid, Octadecansäurechlorid, -anhydrid, Ölsäurechlorid, -anhydrid, Linolsäurechlorid, -anhydrid, Nonadecansäurechlorid, -anhydrid, Eicosansäurechlorid, -anhydrid, Benzoesäurechlorid, 4-Chlor-benzoesäurechlorid, 3,4-Dimethoxybenzoesäurechlorid, Phenylessigsäurechlorid, 3-Phenylpropionsäurechlorid, 4-Phenylbuttersäurechlorid, Chlorameisensäuremethylester, Chlorameisensäureethylester, Chlorameisensäurepropylester, Chlorameisensäureisopropylester, Chlorameisensäureallylester, Chlorameisensäurebutylester, Chlorameisensäurepentylester, Chlorameisensäurehexylester, Chlorameisensäureheptylester, Chlorameisensäureoctylester, Chlorameisensäurenonylester, Chlorameisensäuredecylester, Chlorameisensäureundecylester, Chlorameisensäuredodecylester, Chlorameisensäuretridecylester, Chlorameisensäuretetradecylester, Chlorameisensäurepentadecylester, Chlorameisensäurehexadecylester, Chlorameisensäureheptadecylester, Chlorameisensäureoctadecylester, Chlorameisensäureoleylester, Chlorameisensäurelinolylester, Chlorameisensäurenonadecylester, Chlorameisensäureeicosylester, Chlorameisensäurebenzylester,

Methansulfonsäurechlorid, Ethansulfonsäurechlorid, Propansulfonsäurechlorid, Allylsulfonsäurechlorid, Butansulfonsäurechlorid, p-Toluolsulfonsäurechlorid, Phenylmethansulfonsäurechlorid, Dimethylcarbamidsäurechlorid, Ethylmethylcarbamidsäurechlorid, Diethylcarbamidsäurechlorid.

Als Acylierungsmittel der Formeln IX und X kommen z.B. in Frage:
Methylisocyanat, Methylisothiocyanat, Ethylisocyanat, Ethylisothiocyanat, Propylisocyanat, Propylisothiocyanat, Allylisocyanat, Allylisothiocyanat, Butylisocyanat, Pentylisocyanat, Hexylisocyanat, Heptylisocyanat, Octylisocyanat, Nonylisocyanat, Decylisocyanat, Undecylisocyanat, Dodecylisocyanat, Tridecylisocyanat, Tetradecylisocyanat, Pentadecylisocyanat, Hexadecylisocyanat, Heptadecylisocyanat, Octadecylisocyanat, Oleylisocyanat, Linolylisocyanat, Nonadecylisocyanat, Eicosylisocyanat, Phenylisocyanat, 4-Chlorphenylisocyanat, Phenylisothiocyanat, Benzylisocyanat.

Verbindungen der Formel I werden hergestellt, indem man Verbindungen der Formel II mit $R^8$ = Alkyl nach den üblichen Verfahren, z.B. durch Reaktion mit Natrium- oder Kaliumhydroxid in wässrigen, wässrig-organischen oder organischen Reaktionsmedien, in die entsprechenden Alkalisalze der Formel II ($R^8$ = Alkali) und durch nachfolgenden Zusatz einer Mineralsäure in die Betaine der Formel I überführt, wobei für den Fall, daß $R^3$ ein alkalihydroxidstabiler Rest ist, $R^3$ in den Formeln I und II identisch ist, während für den Fall, daß $R^3$ eine verseifbare Gruppe enthält, $R^3$ in Formel I ein Wasserstoffatom darstellt.

Verbindungen der Formel II können auch hergestellt werden, indem man Verbindungen der Formel V (mit $R^4$, $R^5$ ungleich Wasserstoff) mit Acylierungsmitteln der Formel VIII in Substanz oder in einem indifferenten organischen Lösungsmittel, gegebenenfalls unter Anwendung einer Hilfsbase, reagieren läßt, wobei als Acylierungsmittel sinngemäß auch organische Isocyanate oder Isothiocyanate der Formeln IX und X in Frage kommen, und die entstandenen Verbindungen der Formel XI,

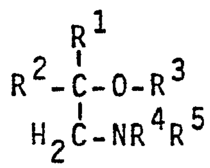

XI

worin $R^1$, $R^2$, $R^3$, $R^4$, $R^5$ die in der Formel II angegebenen Bedeutungen haben, mit einem Alkylierungsmittel der Formel VI in einem indifferenten organischen Lösungsmittel behandelt.

Die vorliegende Erfindung betrifft ebenfalls pharmazeutische Präparate, welche die N-(2-Hydroxyalkyl)-aminosäuren und ihre Derivate der allgemeinen Formel I und II enthalten.

Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Verbindungen liegt üblicherweise zwischen 1 - 1000 mg pro Tag,

vorzugsweise zwischen 10 und 500 mg und kann ein- oder mehrmals, bevorzugt zwei- bis dreimal täglich, verabreicht werden.

Die Herstellung der erfindungsgemäßen Verbindungen wird durch die folgenden Beispiele näher erläutert. Die angegebenen Schmelzpunkte wurden mit einem Büchi-Schmelzpunktbestimmungsapparat gemessen und sind nicht korrigiert. Die IR-Spektren wurden mit dem Gerät Nicolet NIC-3600 aufgenommen.

Die erfindungsgemäßen Verbindungen zeigen in vitro an der Hemmung der Koloniebildung von Zellen der Mäuse-Leukämie L 1210 eine gute proliferationshemmende und zytotoxische Antitumor-Wirksamkeit.

Beispielsweise wird bei obengenannter Versuchsmethode bei einer Konzentration von weniger als 1 µg/ml das Auswachsen von Leukämiezell-Kolonien um mehr als 90 % inhibiert.

Diese antiproliferative, tumorzellwachstumhemmende Wirkung ist mit der Wirkung des bekannten Arzneimittels cis-Platin vergleichbar.

Die niedrigste, bereits proliferationshemmende Konzentration im Versuch ist beispielsweise 1 ng/ml.

Als allgemeiner Konzentrationsbereich für die proliferationshemmende Wirkung (Versuch wie oben) kommt beispielsweise infrage: 1ng/ml bis 10 µg/ml.

Indikationen,für die die erfindungsgemäßen Verbindungen in Betracht kommen können: Neoplasien, maligne Erkrankungen.

Kontraindikationen: Unbekannt, evtl. Schwangerschaft.

Die pharmazeutischen Zubereitungen enthalten im allgemeinen zwischen 25 bis 4000 mg, vorzugsweise 100 bis 1000 mg der erfindungsgemäßen aktiven Komponente(n).

Die Verabreichung kann beispielsweise in Form von Kapseln, Zäpfchen, Salben, Gelees, Cremes oder in flüssiger Form erfolgen. Als flüssige Anwendungsformen kommen zum Beispiel in Frage: Ölige oder alkoholische beziehungsweise wässrige Lösungen sowie Suspensionen und Emulsionen beziehungsweise Lyophilisate. Bevorzugte Anwendungsformen sind Kapseln, die zwischen 100 und 500 mg oder Lösungen, die zwischen 1 bis 20 % an aktiver Substanz enthalten.

Die Einzeldosis der erfindungsgemäßen aktiven Komponenten kann beispielsweise liegen

a) bei oralen Arzneiformen zwischen 0,5 bis 500 mg/kg, vorzugsweise zwischen 5 bis 100 mg/kg,

b) bei parenteralen Arzneiformen (zum Beispiel intravenös, intramuskulär) zwischen 0,1 bis 20 mg/kg, vorzugsweise 1 bis 10 mg/kg,

c) bei Arzneiformen zur lokalen Applikation auf die Haut und Schleimhäute (zum Beispiel in Form von Lösungen, Lotionen, Emulsionen, Salben und so weiter) zwischen 1 und 1000 mg/g, vorzugsweise zwischen 20 und 200 mg/g.

Beispielsweise können 3 mal täglich 1 bis 3 Kapseln mit einem Gehalt von 50 bis 250 mg wirksamer Substanz oder zum Beispiel bei intravenöser Injektion 1 bis 4 mal täglich eine Ampulle von 10 bis 100 ml Inhalt mit 200 bis 1000 mg Substanz empfohlen werden. Bei oraler Verabreichung ist die minimale tägliche Dosis beispielsweise 250 mg; die maximale tägliche Dosis bei oraler Verabreichung soll nicht über 4 g liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation oberhalb 1000 mg/kg, bei 4maliger oraler Applikation an aufeinanderfolgenden Tagen oberhalb einer Gesamtdosis von 2150 mg/kg.

Bei intraperitonealer Verabfolgung an der Maus liegt die akute Toxizität, ausgedrückt durch die LD 50, zwischen 50 und 150 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

Beispiel 1

N-2-Acetyloxyoctadecyl-N,N-dimethyl-3-ethoxycarbonyl-propylammoniumbromid

a) 1-Dimethylamino-octadecan-2-ol
41 g 1,2-Epoxyoctadecan werden in 150 ml Tetrahydrofuran gelöst, 38 ml einer 40 %igen Lösung von Dimethylamin in Wasser hinzugefügt und die Mischung 2 Stunden bei 60°C im geschlossenen Gefäß gerührt. Die Reaktionslösung wird im Vakuum eingeengt und der Rückstand durch Säulenchromatographie (Kieselgel//Chloroform/Methanol/Wasser) gereinigt.
Ausbeute: 39,1 g
IR (in KBr): 3450, 1470 $cm^{-1}$

b) 2-Acetyloxy-1-(N,N-dimethylamino)-octadecan
39 g 1-Dimethylamino-octadecan-2-ol und 27 ml Triethylamin werden in 200 ml absolutem Chloroform gelöst und 13,2 ml Acetylchlorid bei 20°C eingetropft. Die Mischung wird 1 Stunde gerührt, mehrfach mit Wasser gewaschen, über Natriumsulfat getrocknet und das Lösungsmittel im Vakuum eingedampft. Der Rückstand wird durch Säulenchromatographie (Kieselgel//Chloroform/Methanol) gereinigt.
Ausbeute: 39,5 g mit Schmp. 35-36°C
IR (in KBr): 1740 $cm^{-1}$

c) N-2-Acetyloxyoctadecyl-N,N-dimethyl-N-3-ethoxycarbonylpropylammoniumbromid

31,5 g 2-Acetyloxy-1-(N,N-dimethylamino)-octadecan und 17,2 g 4-Brombuttersäureethylester werden in 200 ml Dimethylformamid gelöst und die Mischung 8 Stunden bei 100°C gerührt. Das Lösungsmittel wird im Vakuum abgedampft und der Rückstand durch Säulenchromatographie (Kieselgel//Chloroform/Methanol) gereinigt.

Ausbeute: 38 g mit Schmp. 55-61°C

IR (in KBr): 1736 $cm^{-1}$

Das entsprechende N-2-Acetyloxyoctadecyl-N,N-dimethyl-N-3-ethoxycarbonylpropylammoniumchlorid wird analog aus 2-Acetyloxy-1-(N,N-dimethylamino)-octadecan und 4-Chlorbuttersäureethylester hergestellt.

Beispiel 2

4-(N,N-Dimethyl-N-2-hydroxyoctadecylammonio)-butyrat

33 g N-2-Acetyloxyoctadecyl-N,N-dimethyl-N-3-ethoxycarbonyl-propylammoniumbromid werden in 100 ml Ethanol aufgenommen und eine Lösung von 9,6 g Natriumhydroxid in 150 ml Ethanol/Wasser = 2/1 (V/V) zugetropft. Die Mischung wird 24 Stunden bei Raumtemperatur gerührt, mit 25 %iger Salzsäure auf ca. pH 8 eingestellt, im Vakuum zur Trockne eingeengt und der Rückstand mit Chloroform extrahiert. Die Chloroformlösung wird über Magnesiumsulfat getrocknet und eingeengt. Der Rückstand wird mit Aceton aufgekocht, abgekühlt, abgesaugt und getrocknet.

Ausbeute: 21,2 g mit Schmp. 195-198°C

IR (in KBr): 3370, 1578 $cm^{-1}$

## Beispiel 3

N-2-Acetyloxyoctadecyl-N,N-dimethyl-N-3-hydroxycarbonyl-propylammoniumchlorid

2 g 4-(N,N-Dimethyl-N-2-hydroxyoctadecylammonio)-butyrat werden in 10 ml Chloroform gelöst und 1 g Essigsäureanhydrid hinzugefügt. Die Mischung wird 48 Stunden bei 40°C gerührt, überschüssiges Acetanhydrid durch Zugabe von Wasser und wenig verd. Salzsäure hydrolysiert, die Lösungsmittel im Vakuum eingedampft und der Rückstand durch Säulenchromatographie (Kieselgel//Chloroform/Methanol/Wasser) gereinigt.
Ausbeute: 1,6 g mit Schmp. 184-186°C
IR (in KBr): 1741, 1720 $cm^{-1}$ (Schulter)

## Beispiel 4

4-(N,N-Dimethyl-N-2-oleoyloxyoctadecylammonio)-butyrat

2 g 4-(N,N-Dimethyl-N-2-hydroxyoctadecylammonio)-butyrat werden in 20 ml Chloroform gelöst und nacheinander 1 g Triethylamin und 3 g Ölsäurechlorid hinzugefügt. Die Mischung wird 48 Stunden bei 40°C gerührt, mit gesättigter Natriumchloridlösung gewaschen, im Vakuum zur Trockne eingeengt und der Rückstand durch Säulenchromatographie (Kieselgel//Chloroform/Methanol) gereinigt.
Ausbeute: 0,3 g mit Schmp. 102-104°C
IR (in KBr): 1731, 1584 $cm^{-1}$

Beispiel 5

4-(N,N-Dimethyl-N-2-stearoyloxyoctadecylammonio)-butyrat.
Analog Beispiel 4 aus:
2 g 4-(N,N-Dimethyl-N-2-hydroxyoctadecylammonio)-butyrat in 20 ml Chloroform,
1 g Triethylamin,
3 g Stearoylchlorid
Ausbeute: 0,4 g mit Schmp. 110-113°C
IR (in KBr): 1730, 1585 $cm^{-1}$

Beispiel 6

N,N-Dimethyl-N-3-hydroxycarbonylpropyl-N-2-methylcarbamoyloxyoctadecylammoniumchlorid
2 g 4-(N,N-Dimethyl-N-2-hydroxyoctadecylammonio)-butyrat werden in 10 ml Chloroform gelöst und 1 ml Dimethylformamid sowie 0,57 g Methylisocyanat hinzugefügt. Die Mischung wird 48 Stunden bei 40°C gerührt, wenig verd. Salzsäure zugetropft, die Lösungsmittel im Vakuum eingedampft und der Rückstand durch Säulenchromatographie (Kieselgel//Chloroform/Methanol/Wasser) gereinigt.
Ausbeute: 0,9 g mit Schmp. 170-172°C
IR (in KBr): 1719, 1684 $cm^{-1}$

Beispiel 7

4-/N-(2-Hydroxyoctadecyl)-N-methyl-ammonio/-butyrat

a) 1-Methylamino-octadecan-2-ol

67 g 1,2-Epoxyoctadecan werden in 250 ml 33 %iger ethanolischer Methylaminlösung gelöst und die Lösung 6 Stunden bei 60°C im geschlossenen Gefäß gerührt. Die Reaktionslösung wird im Vakuum eingeengt und der Rückstand aus Ether/Hexan umkristallisiert.

Ausbeute: 45,9 g mit Schmp. 84-86°C

b) N-(2-Hydroxyoctadecyl)-N-(3-ethoxycarbonylpropyl)-N-methylammoniumbromid

25 g 1-Methylamino-octadecan-2-ol und 16,2 g 4-Brombuttersäureethylester werden in 160 ml Dimethylformamid gelöst und die Mischung 5 Stunden bei 60°C gerührt. Das Lösungsmittel wird im Vakuum abgedampft und der Rückstand durch Säulenchromatographie (Kieselgel//Chloroform/Methanol) gereinigt.

Ausbeute: 22,9 g

c) 4-/N-(2-Hydroxyoctadecyl)-N-methyl-ammonio/-butyrat

22,5 g N-(2-Hydroxyoctadecyl)-N-(3-ethoxycarbonylpropyl)-N-methyl-ammoniumbromid werden in 100 ml Ethanol aufgenommen und eine Lösung von 4,3 g Natriumhydroxid in 150 ml Ethanol/Wasser = 2/1 (V/V) zugetropft. Die Mischung wird 24 Stunden bei Raumtemperatur gerührt, mit 25 %iger Salzsäure auf pH 8,5-9,0 eingestellt, im Vakuum zur Trockne eingeengt und der Rückstand mit Ethanol extrahiert. Die ethanolische Lösung wird wiederum eingeengt und der Rückstand mit Chloroform extrahiert, die Chloroformlösung mit Magnesiumsulfat getrocknet und das Lösungsmittel abgezogen. Der Rückstand wird mehrfach mit warmem Aceton gewaschen und getrocknet.

Ausbeute: 14,7 g mit Schmp. 92-95°C

IR (in KBr): 1588 $cm^{-1}$

Beispiel 8

N-(2-Acetyloxyoctadecyl)-N-(3-hydroxycarbonylpropyl)-N-methyl-ammoniumchlorid

1,4 g 4-/N-(2-Hydroxyoctadecyl)-N-methyl-ammonio/-butyrat, 1,3 g Ionenaustauscher (Permutit RSP-120, saure Form), 5 ml Eisessig und 5 ml Essigsäureanhydrid werden gemischt und die Mischung 12 Stunden bei Raumtemperatur gerührt. Der Ionenaustauscher wird abfiltriert, das Filtrat mit 1 ml 25 %iger Salzsäure versetzt, zur Trockne eingeengt und der Rückstand durch Säulenchromatographie (Kieselgel// Chloroform/Methanol) gereinigt.
Ausbeute: 0,9 g mit Schmp. 77-78°C
IR (in KBr): 1745, 1713 $cm^{-1}$

Beispiel 9

4-/N,N-Dimethyl-N-(2-methoxyoctadecyl)-ammonio/-butyrat

1,5 g 4-/N,N-Dimethyl-N-(2-hydroxyoctadecyl)-ammonio/-butyrat werden in 30 ml trockenem Dimethylformamid gelöst und die Lösung nach Zugabe von 2,1 g gepulvertem Kaliumhydroxid und 1,1 g Methyljodid 12 Stunden bei Raumtemperatur gerührt. Das Lösungsmittel wird im Vakuum abgezogen, der Rückstand mit Wasser aufgenommen und mit verd. Salzsäure auf pH 9-9,5 eingestellt. Die Lösung wird eingeengt und der Rückstand mit Chloroform verrührt, die Chloroformlösung filtriert und das Filtrat erneut eingeengt. Das zurückbleibende Rohprodukt wird durch Säulenchromatographie (Kieselgel//Chloroform/Methanol) gereinigt und lyophylisiert (Lösungsmittel: Wasser).
Ausbeute: 0,8 g mit Schmp. 132-134°C

Beispiel (Lyophilisat)

In 3 Liter Wasser für Injektionszwecke werden unter Stickstoffbegasung 500 g Mannit und 100 g Verbindung gemäß Beispiel 2 gelöst und mit Wasser für Injektionszwecke auf 4 Liter aufgefüllt. Diese milchige Dispersion wird durch Ultraschall-Behandlung oder mit Hilfe eines Spalthomogenisators in ein leicht opaleszierendes kolloiddisperses System überführt.
Unter aseptischen Bedingungen wird nun über ein Membranfilter von 0,22 µm Porenweite sterilfiltriert und zu 40 ml in 100 ml Injektionsflaschen unter Stickstoffbegasung abgefüllt. Die Flaschen versieht man mit Gefriertrocknungsstopfen und lyophilisiert in einer geeigneten Anlage. Nach der Trocknung wird mit sterilem, getrocknetem Stickstoff begast und die Flaschen in der Anlage verschlossen. Die Stopfen werden mit einer Bördelkappe gesichert.
Für die intravenöse Anwendung wird das Lyophilisat in 100 ml Wasser für Injektionszwecke rekonstituiert.
1 Flasche enthält 1 g Verbindung gemäß Beispiel 2.

Beispiel (Kapseln)

1,25 kg Verbindung gemäß Beispiel 3 werden in 5 kg Chloroform gelöst und in dieser Lösung 1,25 kg Aerosil suspendiert. Anschließend wird das Lösungsmittel im Vakuum bei 30° C abgezogen.
Die trockene Masse wird durch ein 1 mm-Sieb gegeben und noch einmal im Vakuum bei 30° C getrocknet, um letzte Lösungsmittelrückstände zu entfernen.
Dieses Granulat wird in bekannter Weise auf einer geeigneten Kapselmaschine in Gelatinehartkapseln der Größe 00 zu 500 mg abgefüllt.
Eine Kapsel enthält 250 mg Verbindung gemäß Beispiel 3.

ASTA-Werke Aktiengesellschaft, Chemische Fabrik
Artur-Ladebeck-Straße 128-152, 4800 Bielefeld 14

Titel: Neue N-(2-Hydroxyalkyl)-aminosäuren und ihre Derivate, Verfahren zu ihrer Herstellung und diese enthaltende pharmazeutische Präparate

## Patentansprüche

1. N-(2-Hydroxyalkyl)-aminosäuren und ihre Derivate der allgemeinen Formeln I und II

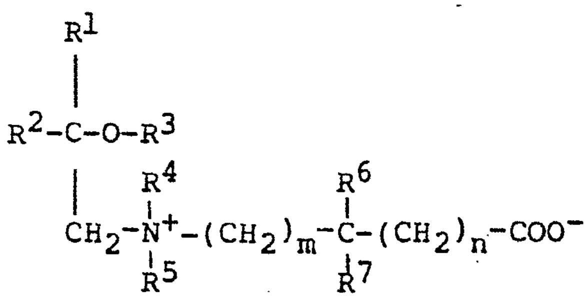

I

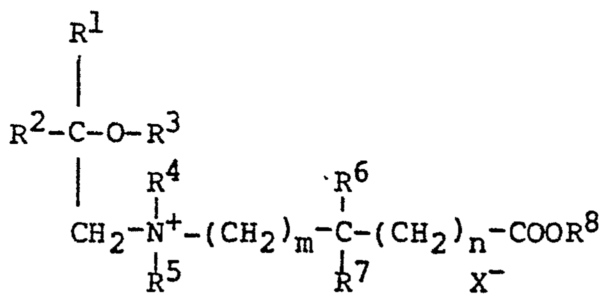

II

worin

$R^1$ einen Alkylrest mit 10-20 Kohlenstoffatomen,

$R^2$ Wasserstoff oder einen Alkylrest mit 1-4 Kohlenstoffatomen,

$R^3$ Wasserstoff, einen gesättigten oder ungesättigten, geradkettigen oder verzweigten Alkylrest mit 1-20 Kohlenstoffatomen, einen Aralkylrest der Formel $-(CH_2)_p-$ 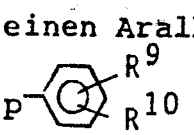

mit p gleich 1-4, $-CO-R^{11}$, $-COOR^{11}$, $-CO-NR^{11}R^{12}$, $-CS-NH-R^{11}$, $-O_2S-R^{11}$,

$R^4$, $R^5$ gleich oder voneinander verschieden sind und Wasserstoff, einen Alkylrest mit 1-4 Kohlenstoffatomen, Phenyl oder Benzyl,

$R^6$, $R^7$ gleich oder voneinander verschieden sind und Wasserstoff, einen linearen oder verzweigten Alkylrest mit 1-4 Kohlenstoffatomen, einen Aralkylrest der Formel $-(CH_2)_q-$ 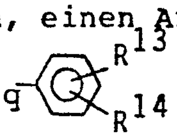

mit q gleich 0-4, eine Methylthioethyl-Gruppe,

$R^8$ Wasserstoff, einen Alkylrest mit 1-6 Kohlenstoffatomen,

$R^9$, $R^{10}$, $R^{13}$, $R^{14}$ gleich oder voneinander verschieden sind und Wasserstoff, Halogen, einen Alkyl- oder Alkoxy-Rest mit 1-4 Kohlenstoffatomen, eine Trifluormethylgruppe oder jeweils zusammen ($R^9+R^{10}$; $R^{13}+R^{14}$) eine Methylendioxygruppe,

$R^{11}$, $R^{12}$ gleich oder voneinander verschieden sind und Wasserstoff, einen gesättigten oder ungesättigten, linearen oder verzweigten Alkylrest mit 1-19 Kohlenstoffatomen, einen Aralkylrest der

Formel $-(CH_2)_r$ 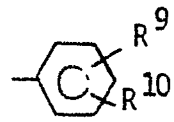 mit r gleich 0-4,

$X^-$ ein Säureanion einer anorganischen oder organischen, ein- oder mehrbasischen Säure und die Summe aus

m + n eine ganze Zahl von 0-10 bedeuten.

2. N-(2-Hydroxyalkyl)-aminosäuren und ihre Derivate der allgemeinen Formeln I und II

worin

$R^1$ einen Alkylrest mit 10-20 Kohlenstoffatomen,

$R^2$ Wasserstoff,

$R^3$ Wasserstoff, einen gesättigten oder ungesättigten Alkylrest mit 1-20 Kohlenstoffatomen, $-CO-R^{11}$, $-COOR^{11}$, $-CO-NR^{11}R^{12}$, $-CS-NH-R^{11}$, $-O_2S-R^{11}$,

$R^4$,$R^5$ gleich oder voneinander verschieden sind und Wasserstoff oder einen Alkylrest mit 1-4 Kohlenstoffatomen,

$R^6$,$R^7$ Wasserstoff,

$R^8$ Wasserstoff oder einen Alkylrest mit 1-6 Kohlenstoffatomen,

$R^{11}$,$R^{12}$ gleich oder voneinander verschieden sind und Wasserstoff, einen gesättigten oder ungesättigten Alkylrest mit 1-19 Kohlenstoffatomen, Phenyl oder Benzyl,

$X^-$ ein Säureanion einer anorganischen oder organischen, ein- oder mehrbasischen Säure und die Summe aus

m + n eine ganze Zahl von 0-10 bedeuten.

3. N-(2-Hydroxyalkyl)-aminosäuren und ihre Derivate der allgemeinen Formeln I und II,

worin

| | |
|---|---|
| $R^1$ | einen Alkylrest mit 10-20 Kohlenstoffatomen, |
| $R^2,R^3,R^6,R^7$ | Wasserstoff, |
| $R^4,R^5$ | Methyl, |
| $R^8$ | Wasserstoff, einen Alkylrest mit 1-6 Kohlenstoffatomen, |
| $X^-$ | ein Säureanion einer anorganischen oder organischen, ein- oder mehrbasischen Säure und die Summe aus |
| m + n | eine ganze Zahl von 0-10 |

bedeuten.

4. N-(2-Hydroxyalkyl)-aminosäuren und ihre Derivate der allgemeinen Formeln I und II,

worin

| | |
|---|---|
| $R^1$ | einen Alkylrest mit 10-20 Kohlenstoffatomen, |
| $R^2,R^6,R^7$ | Wasserstoff, |
| $R^3$ | $-CO-R^{11}$, $-O_2S-CH_3$ |
| $R^4,R^5$ | Methyl, |
| $R^8$ | Wasserstoff, einen Alkylrest mit 1-6 Kohlenstoffatomen, |
| $R^{11}$ | Wasserstoff, einen gesättigten oder ungesättigten Alkylrest mit 1-19 Kohlenstoffatomen, |
| $X^-$ | ein Säureanion einer anorganischen oder organischen, ein- oder mehrbasischen Säure und die Summe aus |
| m + n | eine ganze Zahl von 0-10 |

bedeuten.

5. N-(2-Hydroxyalkyl)-aminosäuren und ihre Derivate der allgemeinen Formeln I und II,

worin

| | |
|---|---|
| $R^1$ | einen Alkylrest mit 10-20 Kohlenstoffatomen, |
| $R^2$,$R^6$,$R^7$ | Wasserstoff, |
| $R^3$ | $-COOR^{11}$, |
| $R^4$,$R^5$ | Methyl, |
| $R^8$ | Wasserstoff, einen Alkylrest mit 1-6 Kohlenstoffatomen, |
| $R^{11}$ | Wasserstoff, einen gesättigten oder ungesättigten Alkylrest mit 1-19 Kohlenstoffatomen, |
| $X^-$ | ein Säureanion einer anorganischen oder organischen, ein- oder mehrbasischen Säure und die Summe aus |
| m + n | eine ganze Zahl von 0-10 |

bedeuten.

6. N-(2-Hydroxyalkyl)-aminosäuren und ihre Derivate der allgemeinen Formeln I und II,

worin

| | |
|---|---|
| $R^1$ | einen Alkylrest mit 10-20 Kohlenstoffatomen, |
| $R^2$,$R^6$,$R^7$ | Wasserstoff, |
| $R^3$ | $-CO-NR^{11}R^{12}$, $-CS-NH-R^{11}$ |
| $R^4$,$R^5$ | Methyl, |
| $R^8$ | Wasserstoff, einen Alkylrest mit 1-6 Kohlenstoffatomen, |
| $R^{11}$ | Wasserstoff, Methyl, Ethyl, |
| $R^{12}$ | einen gesättigten oder ungesättigten Alkylrest mit 1-9 Kohlenstoffatomen, |

$X^-$ ein Säureanion einer anorganischen oder organischen, ein- oder mehrbasischen Säure und die Summe aus

m + n eine ganze Zahl von 0-10 bedeuten.

7. Verbindungen der Formel I oder II, worin

$R^1$ $C_{10}$-$C_{20}$-Alkyl,

$R^2$ Wasserstoff,

$R^3$ Wasserstoff oder $C_2$-$C_6$-Alkanoyl,

$R^4$, $R^5$ $C_1$-$C_6$-Alkyl,

$R^6$, $R^7$ Wasserstoff,

$R^8$ $C_1$-$C_6$-Alkyl oder Wasserstoff,

m die Zahl Null und

n eine Zahl von 1 bis 6, insbesondere 1, 2, 3 oder 4 ist.

8. Verfahren zur Herstellung von Verbindungen der Formel II gemäß den Ansprüchen 1-7, dadurch gekennzeichnet, daß man Epoxide der allgemeinen Formel III,

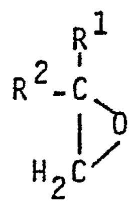

in der $R^1$ und $R^2$ die in den Formeln I und II angegebenen Bedeutungen haben, mit Aminen der Formel IV,

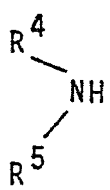

IV

in der $R^4$ und $R^5$ die in den Formeln I und II angegebenen Bedeutungen besitzen, in organischen oder organisch-wässrigen Lösungsmittelsystemen, bevorzugt Methanol oder Ethanol, gegebenenfalls unter Druck, zu den ß-Aminoalkoholen der Formel V,

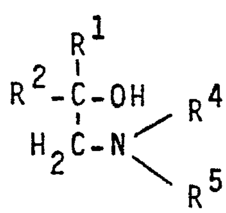

V

in der $R^1$, $R^2$, $R^4$, $R^5$ die in den Formeln I und II angegebenen Bedeutungen haben, umsetzt und diese mit einem Alkylierungsmittel der Formel VI,

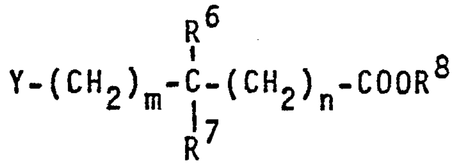

VI

in der $R^6$, $R^7$, $R^8$, m und n die in den Formeln I und II angegebenen Bedeutungen haben und Y ein Chlor-, Brom-, Jodatom oder eine ähnliche Abgangsgruppe darstellt, in einem indifferenten organischen Lösungsmittel behandelt, wobei Verbindungen der Formel II mit $R^3$=H entstehen.

9. Verfahren zur Herstellung von Verbindungen der Formel II mit $R^3$=H gemäß den Ansprüchen 1-7, dadurch gekennzeichnet, daß man Epoxide der allgemeinen Formel III mit Aminocarbonsäureestern der allgemeinen Formel VII,

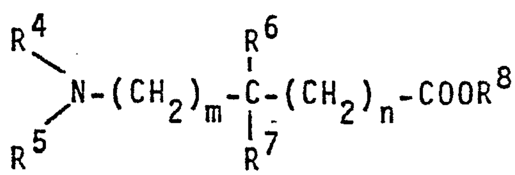

VIII

in der $R^4$, $R^5$, $R^6$, $R^7$, $R^8$, m und n die in den Formeln I und II angegebenen Bedeutungen haben, in organischen oder organisch-wässrigen Lösungsmittelsystemen, bevorzugt Methanol oder Ethanol, umsetzt und die entstandene Stickstoffbase mit einer Säure der Formel II HX, deren Anion $X^-$ die in Formel II angegebene Bedeutung hat, behandelt.

10. Verfahren zur Herstellung von Verbindungen der Formeln I und II gemäß den Ansprüchen 1-7, dadurch gekennzeichnet, daß man Verbindungen der Formeln I und II (mit $R^3$ = Wasserstoff; $R^4$, $R^5$ ungleich Wasserstoff) mit Alkylierungsmitteln oder Acylierungsmitteln der Formel VIII,

$$Z - R^3 \qquad \text{VIII}$$

worin $R^3$ die in den Formeln I und II angegebene Bedeutung hat (mit Ausnahme von Wasserstoff) und Z ein Halogenatom, Acyloxy-Rest oder ähnliche Abgangsgruppe darstellt, in Substanz oder einem indifferenten organischen Lösungsmittel, gegebenenfalls unter Anwendung einer Hilfsbase, die mit dem Acylierungsmittel oder Alkylierungsmittel und dem Rest $R^8$ compatibel ist, umsetzt, wobei als Acylierungsmittel sinngemäß auch organische Isocyanate oder Isothiocyanate der Formeln IX und X in Frage kommen,

$$R^{11} - N = C = O \qquad\qquad R^{11} - N = C = S$$

IX X

in denen $R^{11}$ die in den Formeln I und II angegebene Bedeutung hat.

11. Verfahren zur Herstellung von Verbindungen der Formel I gemäß den Ansprüchen 1-7, dadurch gekennzeichnet, daß man Verbindungen der Formel II nach dem üblichen Verfahren, z.B. durch Reaktion mit einem Alkalihydroxid in wässrigen, wässrig-organischen oder organischen Reaktionsmedien, in die entsprechenden Alkalisalze der Formel II ($R^8$ = Alkali) und durch nachfolgenden Zusatz einer Mineralsäure in die Betaine der Formel I überführt, wobei für den Fall, daß $R^3$ ein

gegenüber Alkalihydroxid stabiler Rest ist, $R^3$ in den Formeln I und II identisch ist, während für den Fall, daß $R^3$ eine verseifbare Gruppe enthält, $R^3$ in der Formel I ein Wasserstoffatom darstellt.

12. Verfahren zur Herstellung von Verbindungen der Formel II mit $R^4$, $R^5 \neq$ H, dadurch gekennzeichnet, daß man Verbindungen der Formel V mit $R^4$, $R^5 \neq$ H mit Acylierungsmitteln der Formel VIII in Substanz oder in einem indifferenten organischen Lösungsmittel, gegebenenfalls unter Anwendung einer Hilfsbase, reagieren läßt, wobei als Acylierungsmittel sinngemäß auch organische Isocyanate oder Isothiocyanate der Formeln IX und X in Frage kommen, und die entstandenen Verbindungen der Formel XI,

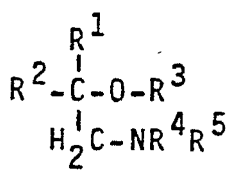

XI

worin $R^1$, $R^2$, $R^3$, $R^4$, $R^5$ die in der Formel II angegebenen Bedeutungen haben, mit einem Alkylierungsmittel der Formel VI in einem indifferenten organischen Lösungsmittel behandelt.

13. Pharmazeutische Präparate, dadurch gekennzeichnet, daß sie eine Verbindung der Formel I oder II gemäß den Ansprüchen 1-7 als Wirkstoff im Gemisch mit üblichen pharmazeutischen Hilfs- und Trägerstoffen enthalten.

14. Verwendung von Verbindungen der Formel I oder II nach einem oder mehreren der vorangegangenen Ansprüche zur Herstellung eines Arzneimittels.